# EUROPEAN PATENT SPECIFICATION

(11) **EP 2 204 106 B1**
(45) Date of publication and mention of the grant of the patent: **16.12.2015**
(21) Application number: 09179869.4
(22) Date of filing: 18.12.2009
(51) Int. Cl.: A45D 33/00, A61K 8/00, A61Q 1/00, B30B 11/00, B65B 1/24

(54) **SELECTIVELY DECORATED COMPACTED MAKE-UP PRODUCT AND PROCESS**
WAHLWEISE DEKORIERTES KOMPAKTIERTES MAKE-UP PRODUKT UND VERFAHREN ZU DESSEN HERSTELLUNG
MAKE-UP COMPACTE ET DECORE SELECTIVEMENT ET PROCEDURE POUR SA PREPARATION

(30) Priority: 23.12.2008 IT MI20082315
(43) Date of publication of application: 07.07.2010
(73) Proprietor: CHROMAVIS S.p.A., 20122 Milan (IT)
(72) Inventor: Priore, Romualdo, 20129 Milano (IT); Bigatto, Guido, 26010 Vaiano Cremasco (IT)
(74) Representative: Vanosi, Adelio Valeriano

(56) References cited:
- EP-A- 0 328 793
- EP-A- 0 432 808
- EP-A- 0 486 394
- EP-A- 1 837 278
- WO-A-2004/103690
- US-A- 5 861 165
- US-A- 6 058 942

## Description

The present invention relates to a make-up product and to a method for its preparation.

Currently, cosmetics such as blushers, eye shadow, foundation creams, in particular compacted powder or extruded cosmetics, have to be surface decorated with detailed regular patterns in order to be made more aesthetically attractive and hence increase their perceived quality level (greater saleability).

Surface decorations are currently applied using various techniques. Some are based on removing material by laser or milling, from a surface previously covered by a light overspray layer. Others use double pressing (dry moulding).

"Dry moulding" is a technique able to form regular detailed patterns but which can penetrate into the product surface only for a maximum depth of 20% (extruded or compacted powders).

Overspray and laser decorations have the drawback of being removed during initial use, whereas dry moulding decorations are slightly more persistent but still remain visible only for a few applications.

EP 1837278 (A1) describes a process for the preparation of a cosmetic product with powders having different features, specifically being multicoloured, carried out by the steps of precipitating cosmetic powders, compressing, forming cavities, and exciding excesses.

An object of the present invention is therefore to provide a make-up product and a method for its preparation which overcomes said drawbacks of the known art, in the sense of providing decorations which remain visible even after many product applications.

These and other objects are attained by a make-up product in accordance with the technical teachings of the accompanying claims.

Further characteristics and advantages of the invention will be apparent from the description of a preferred but non-exclusive embodiment of the make-up product, illustrated by way of non-limiting example in the accompanying drawings, in which:
Figure 1 shows schematically a first step in the preparation of the cosmetic according to the present invention;
Figure 2 shows schematically a second step in the preparation of the make-up product according to the present invention; and
Figures 3 and 4 show schematically alternate third steps of the method of the present invention.

With reference to said figures, these show a section through a make-up product indicated overall by the reference 1 A and 1 B.

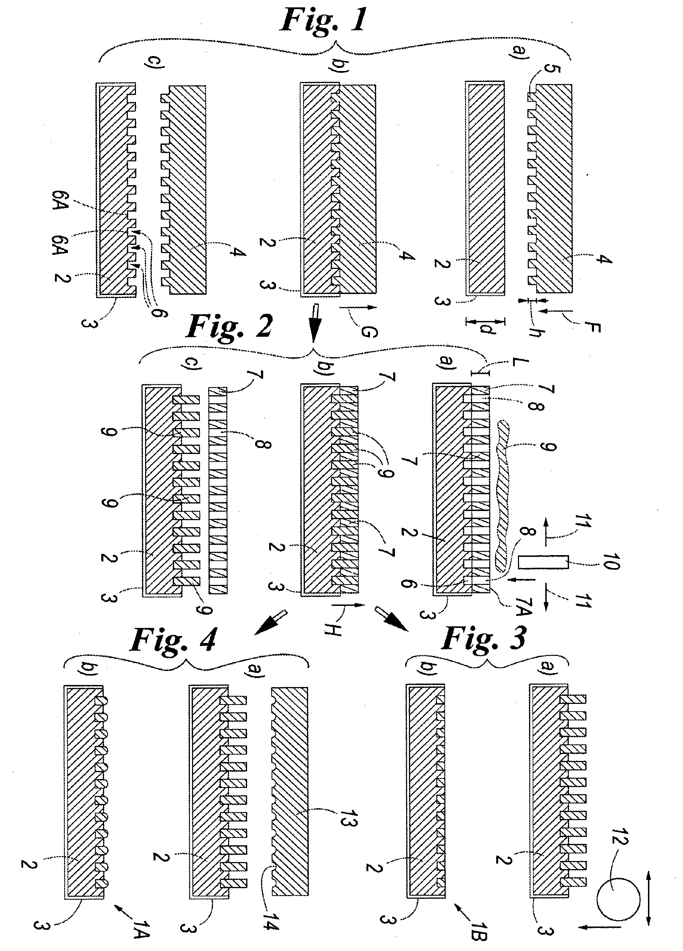

The method for preparing the make-up product in question comprises a first step illustrated in Figure 1. Specifically, a substrate 2 is formed from a first cosmetic product and is housed for convenience within a base 3. The first cosmetic product is advantageously of known compacted powder type, or of the compacted extruded type illustrated for example in EP1458331 in the name of the same applicant.

The substrate 2 is then pressed (Figure 1, arrow F) with a first die 4 comprising one or more reliefs (in positive). The reliefs 5 are disposed on the die such as to represent a decoration. In the example they are disposed such as to form parallel lines which extend in a direction perpendicular to the sheet carrying the drawing. In alternative embodiments, the relief or reliefs can represent the shape of flowers, letters, images or any decorative element. Advantageously the reliefs have a maximum height "h" which is related to the height d of the substrate. In particular the reliefs have a height between 5% and 40% of the height d of the substrate.

When the die 4 has been raised (arrow G), recesses 6 of dimensions and shape substantially identical to the reliefs 5 remain impressed in the substrate; at the bottom 6A of these recesses the first cosmetic product housed in the base is more compacted and hence more dense than in other regions of the substrate. The recesses evidently present the negative shape of the decoration of the reliefs 5.

In the second step, a masking element 7 presenting passages 8 corresponding with the die reliefs 5 is positioned on, and in contact with, the previously worked substrate 2. The masking element is positioned such that the passages 8 correspond with the recesses 6 of the substrate 2. On the upper surface 7A of the masking element 7 a second cosmetic product 9 is then deposited, this being of known type, preferably of cosmetic paste or extruded form (for example that described in IT MI2007A002336 in the name of the same applicant) which is made to penetrate through said passages 8 and is then deposited selectively in the substrate recesses 6. The second product is preferably spread by a spatula 10 which is brought into contact with the surface 7A of the masking element 7 and moved (arrow 11) along this surface so as to cause the second product 9 to penetrate completely into the passages and completely fill them (Figure 2b).

The masking element 7 is then removed (arrow H). The second product 9 hence remains housed in the recesses with a portion of it projecting from the recesses by a height equal to that of the masking element 7 (Figure 2c). The masking element advantageously has a height L between 0.2 and 0.7 mm, which is hence also the height of the second product 9 projecting from the recesses 6.

It is certainly possible to use cosmetic products with different characteristics or colours to fill different passages.

On termination of the aforedescribed process, a finishing procedure is then applied both to those portions of the second product projecting from the substrate and to at least the edge of a surface portion of the substrate 2. Before the crude product of Figure 2c is processed, it can if necessary be dried, with the loss of the volatile component. In particular, drying can continue until the volatile component is brought to below 4% by weight.

By way of example, the procedure can be of two types, the first shown in Figure 3 and the second in Figure 4 which essentially show alternative procedures to be carried out on the crude product of Figure 2c.

In the first alternative, the procedure comprises removal of the excess substrate portion (second product) by rubbing on an abrasive surface 12 to hence obtain a homogeneous smooth surface (Figure 3B).

A further compacting process can then be carried out in traditional manner by means of a flat die, or by using a concave die which gives the cosmetic a convex surface in relation to the decorations.

The alternative procedure, shown in Figure 4, uses a second die 13. This second die 13 presents a series of profiled grooves 14, each in a position substantially corresponding to the position of the recesses 6 and arranged to compact and shape, by giving it a predetermined shape, a portion of said second product projecting from the substrate 2. In this manner the projecting portions of the second product can be compacted, rounded or shaped at will, to further increase the appeal of the product.

The pressure of the second die 13 on the substrate also slightly compacts the substrate surface.

Essentially, by means of the described process a cosmetic is obtained comprising a substrate formed from a first cosmetic product which presents on its surface at least one recess 6 which is at least partially filled with a second cosmetic product.

Advantageously the second product hence forms a decoration on the substrate surface which involves not only its surface, but is present in depth within the substrate (up to about 5-40% of its height), the decoration hence remaining well visible and defined even after several applications of the cosmetic.

The second cosmetic product can also project from the recess to form a relief decoration on the substrate surface.

Advantageously the first and second cosmetic product can differ by their intrinsic characteristics and offer completely different cosmetic performances.

By way of example, the first product could be matt and printable and the second product be pearlescent and covering. In particular, the printing/covering capacity, the final effect (matt, pearlescent, satinized), the volumes (flat, in slight relief, in high relief, hollow), and the colours could be varied between one and the other product.

## Claims

1. A method for preparing a surface-decorated make-up product (1 A, 1 B), comprising the steps of:
a. preparing a substrate (2) formed from a first cosmetic product
b. pressing a first die (4) comprising at least one relief onto said substrate to form at least one recess (6) in said substrate
c. removing the first die
d. disposing on the substrate a masking element (7) presenting at least one passage (8) in a position corresponding with at least one relief of the die and hence corresponding with at least one recess (6) of the substrate
e. causing at least one second cosmetic product (9) to penetrate into said at least one recess of said substrate, and
f. removing the masking element
g. carrying out a processing procedure on a portion of said second cosmetic product projecting from the substrate and on a surface portion of the substrate.

2. A method as claimed in claim 1, **characterised in that** said processing procedure consists of removing a surface portion of said substrate (2) and all that part of said second product projecting from the substrate in order to shave the surface of the cosmetic product.

3. A method as claimed in one or more of the preceding claims, **characterised in that** said processing procedure comprises the step of modelling with a second die (13) the projecting portion of the second product and at least partially compacting the surface of the substrate.

4. A method as claimed in one or more of the preceding claims, **characterised in that** said second die (13) presents a series of profiled grooves (14), each arranged to compact and model a portion of said second product projecting from the substrate.

5. A method as claimed in one or more of the preceding claims, **characterised in that** said second product (9) is pushed to penetrate into said at least one passage such as to fill it completely, by spreading it onto the upper surface of the second die.

6. A method as claimed in one or more of the preceding claims, **characterised in that** after said processing procedure, the make-up product is further compacted.

7. A method as claimed in one or more of the preceding claims, **characterised in that** the reliefs of said second die are disposed such as to present a decoration to be reproduced on the surface of the make-up product.

8. A method as claimed in one or more of the preceding claims, **characterised in that** before carrying out said processing procedure, the two cosmetic products are dried with the loss of at least one volatile component.

9. A method as claimed in one or more of the preceding claims, **characterised in that** the first cosmetic product is a compacted powder and/or a compacted extrusion and the second cosmetic product is a blend and/or an extrusion.

10. A method as claimed in one or more of the preceding claims, **characterised in that** said masking element has a height between 0.2 and 0.7 cm.

11. A method as claimed in one or more of the preceding claims, **characterised in that** said recess involves up to 40% of the height of said substrate.

12. A make-up product comprising a substrate formed from a first cosmetic product (2), **characterised by** presenting on the surface of said first cosmetic product at least one recess at least partially filled with a second cosmetic product (9).

13. A make-up product as claimed in the preceding claim, **characterised in that** at the bottom of said recess (6A) the substrate has a density greater than the density of the same substrate in a position in which said recess is not present.

14. A make-up product as claimed in one or more of the preceding claims, **characterised in that** said second product (9) projects from said recess.

15. A make-up product as claimed in one or more of the preceding claims, **characterised in that** said second product forms a decoration on the surface of said substrate.

16. A make-up product or method as claimed in one or more of the preceding claims, **characterised in that** said first and second product differ from each other by a chromatic characteristic.

## Patentansprüche

1. Verfahren zur Herstellung eines oberflächendekorierten Make-up Produkts (1A, 1B), das die folgenden Schritte umfasst:
a. Herstellung eines aus einem ersten Kosmetikprodukt gebildeten Substrats (2)
b. Druck einer mindestens einen Vorsprung umfassenden ersten Matrize (4) auf dem genannten Substrat, um mindestens eine Ausnehmung (6) in dem genannten Substrat zu bilden
c. Entfernung der ersten Matrize
d. Anordnung auf das Substrat eines Maskierungselements (7), das mindestens einen Durchgang (8) in einer mindestens einem Vorsprung der Matrize entsprechenden und demzufolge mindestens einer Ausnehmung (6) des Substrats entsprechenden Stellung aufweist
e. Verursachung des Eindringens mindestens eines zweiten Kosmetikprodukts (9) in die genannte mindestens eine Ausnehmung des genannten Substrats, und
f. Entfernung des Maskierungselements
g. Durchführung eines Verarbeitungsverfahrens auf einer aus dem Substrat hervorragenden Portion des genannten Kosmetikprodukts sowie auf einer Oberflächenportion des Substrats.

2. Verfahren nach Anspruch 1, **dadurch gekennzeichnet, dass** das genannte Verarbeitungsverfahren aus der Entfernung einer Oberflächenportion des genannten Substrats (2) sowie der ganzen aus dem Substrat hervorragenden Portion des genannten zweiten Produkts besteht, um die Oberfläche des Kosmetikprodukts auszugleichen.

3. Verfahren nach einem oder mehreren der vorangegangenen Ansprüche, **dadurch gekennzeichnet, dass** das genannte Verarbeitungsverfahren die Modellierung der hervorragenden Portion des zweiten Produkts mit einer zweiten Matrize (13) und die mindestens teilweise Kompaktierung der Oberfläche des Substrats umfasst.

4. Verfahren nach einem oder mehreren der vorangegangenen Ansprüche, **dadurch gekennzeichnet, dass** die genannte zweite Matrize (13) eine Reihe von profilierten Nuten (14) aufweist, jede derart angeordnet, dass eine Portion des genannten aus dem Substrat hervorragenden zweiten Produkts kompaktiert und modelliert wird.

5. Verfahren nach einem oder mehreren der vorangegangenen Ansprüche, **dadurch gekennzeichnet, dass** das genannte zweite Produkt (9) derart gedrückt wird, dass es in den genannten mindestens einen Durchgang eindringt und ihn ganz ausfüllt, indem es sich auf der oberen Oberfläche der zweiten Matrize ausbreitet.

6. Verfahren nach einem oder mehreren der vorangegangenen Ansprüche, **dadurch gekennzeichnet, dass** nach dem genannten Verarbeitungsverfahren das Make-up Produkt weiter kompaktiert wird.

7. Verfahren nach einem oder mehreren der vorangegangenen Ansprüche, **dadurch gekennzeichnet, dass** die Vorsprünge der genannten zweiten Matrize derart angeordnet sind, dass sie eine auf der Oberfläche des Make-up Produkts wiederzugebende Dekoration aufweisen.

8. Verfahren nach einem oder mehreren der vorangegangenen Ansprüche, **dadurch gekennzeichnet, dass**, bevor das genannte Verarbeitungsverfahren durchgeführt wird, die zwei Kosmetikprodukte getrocknet werden, indem sie einen flüchtigen Stoff verlieren.

9. Verfahren nach einem oder mehreren der vorangegangenen Ansprüche, **dadurch gekennzeichnet, dass** das erste Kosmetikprodukt ein kompaktiertes Pulver und/oder eine kompaktierte Extrusion ist und das zweite Kosmetikprodukt eine Mischung und/oder eine Extrusion ist.

10. Verfahren nach einem oder mehreren der vorangegangenen Ansprüche, **dadurch gekennzeichnet, dass** das genannte Maskierungselement eine Höhe zwischen 0,2 und 0,7 cm hat.

11. Verfahren nach einem oder mehreren der vorangegangenen Ansprüche, **dadurch gekennzeichnet, dass** die genannte Ausnehmung bis zu 40% der Höhe des genannten Substrats einnimmt.

12. Ein Make-up Produkt umfassend ein aus einem ersten Kosmetikprodukt (2) gebildete Substrat, **dadurch gekennzeichnet, dass** es an der Oberfläche des genannten ersten Kosmetikprodukts mindestens eine mindestens teilweise mit einem zweiten Kosmetikprodukt (9) ausgefüllte Ausnehmung aufweist.

13. Ein Make-up Produkt nach dem vorangegangenen Anspruch, **dadurch gekennzeichnet, dass** am Boden der genannten Ausnehmung (6A) das Substrat eine höhere Dichte hat als die Dichte desselben Substrats in einer Stellung, worin die genannte Ausnehmung nicht vorhanden ist.

14. Ein Make-up Produkt nach einem oder mehreren der vorangegangenen Ansprüche, **dadurch gekennzeichnet, dass** das genannte zweite Produkt (9) aus der genannten Ausnehmung hervorragt.

15. Ein Make-up Produkt nach einem oder mehreren der vorangegangenen Ansprüche, **dadurch gekennzeichnet, dass** das genannte Produkt eine Dekoration auf der Oberfläche des genannten Substrats bildet.

16. Ein Make-up Produkt oder Verfahren nach einem oder mehreren der vorangegangenen Ansprüche, **dadurch gekennzeichnet, dass** das genannte erste Produkt und das genannte zweite Produkt sich in einem farbigen Merkmal voneinander unterscheiden.

## Revendications

1. Méthode pour préparer un make-up (lA, 1B) décoré en surface, comprenant les passages de:
a. préparer un substrat (2) formé par un premier produit cosmétique
b. presser une première matrice (4) comprenant au moins un relief sur ledit substrat pour former au moins un renfoncement (6) dans ledit substrat
c. retirer la première matrice
d. agencer sur le substrat un élément de masquage (7) présentant au moins un passage (8) dans une position correspondante à au moins un relief de la matrice et donc correspondante à au moins un renfoncement (6) du substrat
e. laisser pénétrer au moins un second produit cosmétique (9) dans ledit renfoncement dudit substrat, et
f. retirer l'élément de masquage
g. exécuter une procédure de traitement sur une portion dudit second produit cosmétique faisant saillie du substrat et sur une portion de surface du substrat.

2. Méthode selon la revendication 1, **caractérisée en ce que** ladite procédure de traitement consiste à retirer une portion de surface dudit substrat (2) et toute la partie dudit second produit qui fait saillie du substrat pour lisser la surface du produit cosmétique.

3. Méthode selon une ou plusieurs des revendications précédentes, **caractérisée en ce que** ladite procédure de traitement comprend le passage de modeler par une seconde matrice (13) la portion saillante du second produit et de compacter au moins partiellement la surface du substrat.

4. Méthode selon une ou plusieurs des revendications précédentes, **caractérisée en ce que** ladite seconde matrice (13) présente une série de rainures profilées (14), chacune agencée pour compacter et modeler une portion dudit second produit saillant du substrat.

5. Méthode selon une ou plusieurs des revendications précédentes, **caractérisée en ce que** ledit second produit (9) est poussé jusqu'à pénétrer dans ledit au moins un passage, de façon à le remplir complètement, en l'étalant sur la surface supérieure de la seconde matrice.

6. Méthode selon une ou plusieurs des revendications précédentes, **caractérisée en ce que**, après ladite procédure de traitement, le make-up est de nouveau compacté.

7. Méthode selon une ou plusieurs des revendications précédentes, **caractérisée en ce que** les reliefs de ladite seconde matrice sont agencés de façon à présenter une décoration à reproduire sur la surface du make-up.

8. Méthode selon une ou plusieurs des revendications précédentes, **caractérisée en ce que**, avant d'exécuter ladite procédure de traitement, les deux produits cosmétiques sont séchés moyennant la perte d'au moins un composant volatile.

9. Méthode selon une ou plusieurs des revendications précédentes, **caractérisée en ce que** le premier produit cosmétique est une poudre compactée et/ou une extrusion compactée, alors que le second produit cosmétique est un mélange et/ou une extrusion.

10. Méthode selon une ou plusieurs des revendications précédentes, **caractérisée en ce que** ledit élément de masquage a une hauteur entre 0,2 2 et 0,7 cm.

11. Méthode selon une ou plusieurs des revendications précédentes, **caractérisée en ce que** ledit renfoncement concerne jusqu'à 40% de la hauteur dudit substrat.

12. Make-up comprenant un substrat formé par un premier produit cosmétique (2), **caractérisé en ce qu'**il présente sur la surface dudit premier produit cosmétique au moins un renfoncement au moins partiellement rempli par un second produit cosmétique (9).

13. Make-up selon la revendication précédente, **caractérisé en ce que** sur le fond dudit renfoncement (6A) le substrat a une densité supérieure à la densité du même substrat dans une position où ledit renfoncement n'est pas présent.

14. Make-up selon une ou plusieurs des revendications précédentes, **caractérisé en ce que** ledit second produit (9) fait saillie dudit renfoncement.

15. Make-up selon une ou plusieurs des revendication précédentes, **caractérisé en ce que** ledit second produit forme une décoration sur la surface dudit substrat.

16. Make-up ou méthode selon une ou plusieurs des revendications précédentes, **caractérisés en ce que** ledit premier et ledit second produit sont différents l'un de l'autre en une caractéristique chromatique.
